# EUROPÄISCHE PATENTSCHRIFT

(11) **EP 3 116 850 B1**
(45) Veröffentlichungstag und Bekanntmachung des Hinweises auf die Patenterteilung: **07.03.2018**
(21) Anmeldenummer: 15709173.7
(22) Anmeldetag: 11.03.2015
(51) Int. Cl.: C07C 209/16, C07C 211/10, C07C 209/48, C07C 209/84, C07C 213/04, C07C 253/08, C07C 253/30

(54) **VERFAHREN ZUR DESTILLATIVEN REINIGUNG VON EDA**
METHOD FOR CLEANING EDA USING DISTILLATION
PROCÉDÉ DE NETTOYAGE PAR DISTILLATION D'EDA

(30) Priorität: 13.03.2014 EP 14159511
(43) Veröffentlichungstag der Anmeldung: 18.01.2017
(73) Patentinhaber: BASF SE, 67056 Ludwigshafen (DE)
(72) Erfinder: LUYKEN, Hermann, 67056 Ludwigshafen (DE); JAEGLI, Stephanie, 67071 Ludwigshafen (DE); LORENZ, Michael, 67061 Ludwigshafen (DE); BRASCHE, Gordon, 60594 Frankfurt (DE); JEGELKA, Markus, 68219 Mannheim (DE); BECKER, Barbara, 69509 Mörlenbach (DE); BAUMANN, Robert, 68529 Mannheim (DE); MELDER, Johann-Peter, 67459 Böhl-Iggelheim (DE); BUSCHHAUS, Boris, 69117 Heidelberg (DE); KRUG, Thomas, 67550 Worms (DE)
(74) Vertreter: BASF IP Association
(86) Internationale Anmeldenummer: PCT/EP2015/055028
(87) Internationale Veröffentlichungsnummer: WO 2015/135971

(56) Entgegenhaltungen:
- WO-A1-2011/067226
- WO-A1-2012/087553
- WO-A2-2010/042168
- DE-B- 1 258 413

## Beschreibung

Die vorliegende Erfindung betrifft ein Verfahren zur destillativen Reinigung von Ethylendiamin (EDA), wobei das Verfahren die Schritte a) und b) umfasst. In Schritt a) wird ein Gemisch (G1) enthaltend Wasser, EDA und N-Methylethylendiamin (N-MeEDA) in eine Destillationsvorrichtung (D1) eingespeist, aus der über Kopf bei einem Druck von größer als 4,8 bara die Hauptmenge des im Gemisch (G1) enthaltenen Wassers abgetrennt wird. Aus dem Sumpf von (D1) wird das an Wasser abgereicherte Gemisch (G2) in Schritt b) in eine Destillationsvorrichtung (D2) eingespeist. Über den Kopf von (D2) wird die Hauptmenge an N-MeEDA abdestilliert. Der aus dem Sumpf von (D2) gewonnene Strom (S3) enthält EDA, wobei die im Gemisch (G1) enthaltenen Komponenten Wasser sowie N-MeEDA weitgehend oder vollständig abgereichert sind. Gegebenenfalls können weitere Destillationsschritte durchgeführt werden, um reines EDA zu erhalten, beispielsweise wenn im Gemisch (G1) zusätzlich Diethylentriamin (DETA) enthalten ist. Sofern im Gemisch (G1) zusätzlich Ammoniak enthalten ist, erfolgt im erfindungsgemäßen Verfahren vorzugsweise vor Durchführung des Schrittes a) zusätzlich eine Ammoniakabtrennung.

Herstellungsverfahren von EDA sind seit Langem bekannt, häufig wird hierzu Blausäure als eines der Edukte eingesetzt. WO 2008/104578 offenbart ein Verfahren zur Herstellung eines Ethylenamingemisches, das EDA enthält, wobei Roh-Aminoacetonitril (Roh-AAN), das weitgehend frei von Formaldehydcyanhydrin ist, bei einer Temperatur von 50 bis 150 °C erhitzt wird. Dabei wird ein Aminonitrilgemisch erhalten, das AAN und Iminodiacetonitril (IDAN) enthält. Dieses Gemisch wird anschließend in Gegenwart eines Katalysators unter Erhalt von EDA und Diethylentriamin (DETA) hydriert. WO 2008/104578 kann weiterhin entnommen werden, dass das eingesetzte Roh-AAN durch Umsetzung eines wässrigen Gemisches von Ammoniak mit FACH im Molverhältnis ≥ 4 : 1 [Mol/Mol] bei einer Temperatur von 50 bis 80 °C erhalten werden kann.

Die Herstellung von Formaldehydcyanhydrin (FACH) ist ebenfalls seit Langem bekannt. Detaillierte Informationen zur Herstellung von FACH aus Formaldehyd und Blausäure können beispielsweise WO 2008/104579 entnommen werden. In diesem Verfahren wird das FACH jedoch nicht zur Herstellung von EDA über AAN durch Umsetzung mit Ammoniak eingesetzt, stattdessen wird dort FACH mit EDA unter Erhalt von Ethylendiamindiacetonitril (EDDN) umgesetzt. EDDN kann wiederum zu Triethylentetraamin (TETA) hydriert werden.

WO 2011/067226 betrifft ein Verfahren zur Destillation eines Gemisches, enthaltend Wasser, Ethylendiamin und N-Methylethylendiamin, wobei das Gemisch in eine Destillationskolonne eingeleitet wird, die bei einem Kopfdruck von 10 mbar bis 4 bar betrieben wird. In diesem Gemisch müssen Wasser und Ethylendiamin in einem speziellen Verhältnis vorliegen. Das zur Destillation verwendete Gemisch ist wiederum ein Reaktionsaustrag, der durch Umsetzung von Formaldehyd, Blausäure, Ammoniak und Wasserstoff oder durch Umsetzung von Ethylenoxid mit Ammoniak unter Ausbildung von Ethanolamin sowie der weiteren Umsetzung von Ethanolamin mit Ammoniak erhalten werden kann.

Aus WO 2011/067226, Seite 2, Zeilen 13 bis 14, ist weiterhin bekannt, dass EDA und N-MeEDA bei Normaldruck ein engsiedendes azeotropes Gemisch bilden, welches sich im Allgemeinen mit technisch vertretbarem Aufwand nicht trennen lässt. In WO 2011/067226 erfolgt die Abtrennung von N-Methylethylendiamin von Ethylendiamin durch Abtrennung eines N-Methylethylendiamin / Wasser-Azeotrops über Kopf einer Kolonne. Im Verfahren gemäß WO 2011/067226 muss in einigen Ausführungsformen sogar noch Wasser zusätzlich in das Verfahren eingespeist werden, um die azeotrope Abtrennung des N-MeEDA/Wasser-Gemisches zu ermöglichen. In der entsprechenden Kolonne verbleiben im Kolonnensumpf immer größere Mengen an Wasser, die in nachfolgenden Destillationsschritten zusätzlich vom Zielprodukt EDA abgetrennt werden müssen.

Weiterhin ist aus DE-A 1 258 413 bekannt, dass EDA mit Wasser bei Atmosphärendruck ein azeotropes Gemisch bildet, das einen Siedepunkt von ca. 118°C aufweist. Das Azeotrop besteht zu 82 % aus EDA und zu 18 % aus Wasser und siedet etwa 2 °C höher als reines EDA (116 °C). Mit steigendem Druck steigt der EDA-Gehalt des Azeotrops. Oberhalb von 4,8 bar existiert kein Azeotrop mehr.

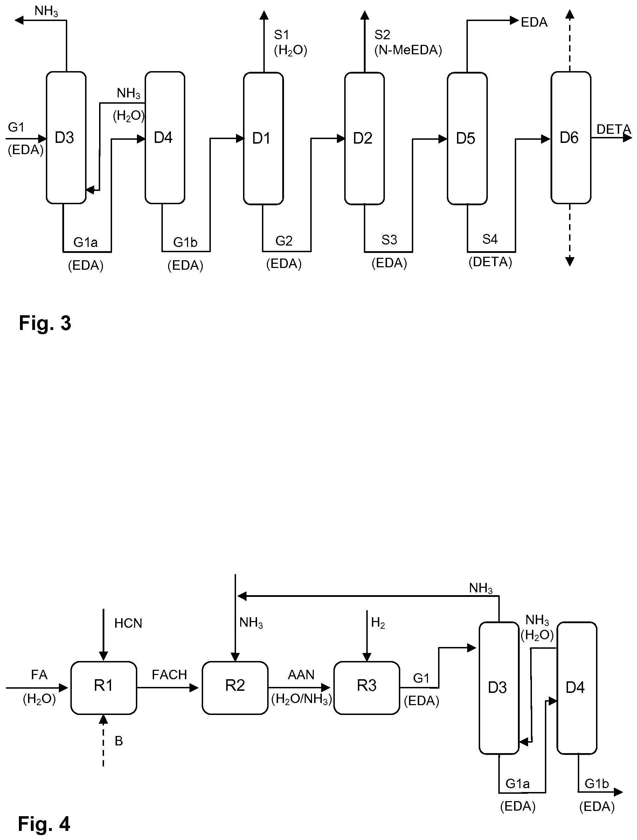

In WO2010/042168 ist ein Verfahren zur Herstellung von Ethylendiamin ausgehend von Ethylenoxid und Ammoniak über das Zwischenprodukt Ethanolamin beschrieben. Das bei der Umsetzung von Ethanolamin mit Ammoniak gebildete Roh-Ethylendiamin enthält die Nebenprodukte N-Ethyl- und N-Methyl-Ethylendiamin. In Beispiel 3 und Figur 4 wird gezeigt, wie man die beiden N-Alkylethylendiamine durch Azeotropdestillation von Ethylendiamin abtrennen kann.

Nachteile bei den Verfahren gemäß WO 2010/042168 und WO 2011/067226 bestehen darin, dass bei den Azeotropdestillationen nicht unerhebliche Mengen an Abwasser anfallen, deren darin enthaltenen Stickstoffverbindungen z.B. in einer Kläranlage abgebaut werden müssen.

Das in WO 2011/067226 beschriebene Verfahren zur Trennung von Ethylendiamin und N-Methylethylendiamin besitzt insbesondere dann Nachteile, wenn das Verhältnis von Wasser zu Ethylendiamin im Zulaufstrom niedriger liegt als im binären Azeotrop und das Gemisch viel N-Methylethylendiamin enthält. In diesem Fall muss dem Zulaufstrom der Kolonne zusätzliches Wasser zugemischt werden (siehe WO 2011/067226, Beispiel 4). Da dieses Wasser wieder abgetrennt werden muss, ist diese Ausführungsform weniger günstig.

Ein weiterer Nachteil besteht darin, dass der Wassergehalt vor der Destillation genau eingestellt werden muss, um EDA-Verluste über Kopf zu vermeiden.

Die der vorliegenden Erfindung zugrunde liegende Aufgabe besteht somit in der Bereitstellung eines neuen Verfahrens zur Reinigung von Ethylendiamin (EDA).

Gelöst wird die Aufgabe durch ein Verfahren zur destillativen Reinigung von Ethylendiamin (EDA), umfassend die Schritte a) und b) mit:
a) Einspeisung eines Gemisches (G1) enthaltend Wasser, EDA und N-Methylethylendiamin (N-MeEDA) in eine Destillationsvorrichung (D1), wobei aus (D1)
   i) über Kopf bei einem Druck von größer als 4,8 bara ein Strom (S1) enthaltend Wasser abdestilliert wird, und
   ii) aus dem Sumpf ein Gemisch (G2) abgezogen wird, wobei das Gemisch (G2) gegenüber dem Gemisch (G1) an Wasser abgereichert ist,
b) Einspeisung des Gemisches (G2) in eine Destillationsvorrichtung (D2), wobei aus (D2)
   i) über Kopf ein Strom (S2) enthaltend N-MeEDA abdestilliert wird, und
   ii) aus dem Sumpf ein Strom (S3) abgezogen wird, wobei der Strom (S3) gegenüber dem Gemisch (G2) an N-MeEDA abgereichert ist.

Ein wesentlicher Vorteil des erfindungsgemäßen Verfahrens ist darin zu sehen, dass keine azeotropen Trennungen von Wasser mit N-MeEDA und/oder mit EDA durchgeführt werden müssen. Stattdessen wird erfindungsgemäß zunächst in einem ersten Verfahrensschritt die Hauptmenge des im eingesetzten Gemisch enthaltenen Wassers von EDA abgetrennt, ohne dass eine azeotrope Wasserabtrennung erfolgt. In einem zweiten Verfahrensschritt wird anschließend N-MeEDA von EDA abgetrennt, wobei wiederum keine azeotropen Bedingungen vorliegen.

Ein weiterer Vorteil des erfindungsgemäßen Verfahrens ist darin zu sehen, dass das Verfahren sehr variabel durchgeführt werden kann. Das aufzureinigende EDA ist unabhängig vom konkreten Herstellungsweg einsetzbar, prinzipiell kann EDA nach allen dem Fachmann bekannten Verfahren hergestellt werden. Beispielsweise kann die EDA-Herstellung ausgehend von Formaldehyd und Blausäure oder unter Verwendung von Ethylenoxid und Ammoniak erfolgen. Bei den aus dem Stand der Technik bekannten Verfahren fallen merkliche Mengen an Abwasser an. Aufgrund der azeotropen Abtrennungsbedingungen enthält das abgetrennte Wasser in der Regel einen großen Anteil an Stickstoffverbindungen als Nebenkomponenten (also EDA, DETA, N-MeEDA etc.). Dieses Abwasser muss daher zusätzlich in einer Kläranlage gereinigt werden.

Erfindungsgemäß wird zwar auch Wasser abgetrennt. Sofern EDA aus wässrigem, 20 bis 60 %-igem Formaldehyd hergestellt wird, sind im aufzutrennenden Gemisch (G1) in der Regel sogar relativ große Mengen an Wasser enthalten, das abgetrennt werden muss. Aufgrund der Verfahrensbedingungen in Schritt a) des erfindungsgemäßen Verfahrens sind im abgetrennten Wasser jedoch sehr wenige stickstoffhaltige Verbindungen enthalten, so dass der Reinigungsaufwand geringer ist.

Ein weiterer Vorteil des erfindungsgemäßen Verfahrens ist darin zu sehen, dass das in Schritt b) als Kopfprodukt anfallende N-MeEDA, das auch als Gemisch mit EDA vorliegen kann, als Komponente zur Herstellung von Epoxidharzen in vorteilhafter Weise einzusetzen ist.

Sofern das im erfindungsgemäßen Verfahren im Gemisch (G1) enthaltene EDA unter Verwendung von Blausäure hergestellt wird, die vollständig frei oder weitgehend frei von Schwefeldioxid (SO₂) ist, ist ein weiterer Vorteil des erfindungsgemäßen Verfahrens darin zu sehen, dass die Standzeit des Hydrierkatalysators (also des Katalysators, der in Schritt A3) gemäß Verfahren A eingesetzt wird) verbessert wird. Dieser Effekt tritt insbesondere dann ein, wenn die zur FACH-Herstellung gemäß Schritt A1) eingesetzte Blausäure vollständig oder zumindest weitgehend frei von Schwefeldioxid sowie gegebenenfalls von weiteren sauren Stabilisatoren wie Schwefelsäure oder Phosphorsäure ist.

Die Verwendung von (weitgehend) SO₂-freier Blausäure in Schritt A3) wirkt sich somit insbesondere positiv auf das Leistungsvermögen des in Schritt A3) eingesetzten Hydrierkatalysators aus. Die AAN-Hydrierung kann somit über lange Zeiten mit hohen EDA-Ausbeuten ohne wesentliches Absinken der Katalysator-Aktivität durchgeführt werden. Insbesondere SO₂ neigt dazu, unter den bei einer Nitrilhydrierung gängigen Verfahrensbedingungen in Gegenwart des Katalysators zu disproportionieren, wobei unter anderem Sulfide entstehen, die wesentlich zur Verminderung des Leistungsvermögens eines Hydrierkatalysators beitragen. Eine solche Disproportionierung wird hingegen bei der Verwendung von Schwefelsäure anstelle von SO₂ nicht oder nur deutlich verringert festgestellt.

Nachfolgend wird die vorliegende Erfindung weiter präzisiert.

Im erfindungsgemäßen Verfahren erfolgt in Schritt a) die Einspeisung eines Gemisches (G1) enthaltend Wasser, EDA und N-Methylethylendiamin (N-MeEDA) in eine Destillationsvorrichung (D1), wobei aus (D1) i) über Kopf bei einem Druck von größer als 4,8 bara ein Strom (S1) enthaltend Wasser abdestilliert wird, und aus ii) dem Sumpf ein Gemisch (G2) abgezogen wird, wobei das Gemisch (G2) gegenüber dem Gemisch (G1) an Wasser abgereichert ist.

Die in Schritt a) im Gemisch (G1) enthaltenen Komponenten Wasser, EDA und N-MeEDA als solche, sind dem Fachmann bekannt. In einer Ausführungsform der vorliegenden Erfindung enthält das Gemisch (G1) im Wesentlichen, vorzugsweise mindestens 99 Gew.-% EDA, N-MeEDA und Wasser. Wie nachfolgend weiter präzisiert, können im Gemisch (G1) auch noch weitere Komponenten wie Diethylentriamin (DETA) oder Ammoniak (NH₃) enthalten sein. Solche zusätzlichen Komponenten werden vorzugsweise (wie nachfolgend ebenfalls präzisiert) entweder vor Durchführung des Schrittes a) - wie im Fall von Ammoniak - oder im Anschluss an den Schritt b) - wie im Fall von DETA - aus dem entsprechenden Gemisch abgetrennt.

Die einzelnen Komponenten des Gemisches (G1) können prinzipiell in beliebigen Verhältnissen zueinander vorliegen.

In einer Ausführungsform der vorliegenden Erfindung enthält das Gemisch (G1) im Wesentlichen, vorzugsweise zu mindestens 99 Gew.-%, insbesondere zu mindestens 99,5 Gew.-%, EDA, N-MeEDA, DETA, Wasser und NH₃. Bei dieser Ausführungsform wird der Ammoniak vorzugsweise vor Durchführung des erfindungsgemäßen Schrittes a) aus dem Gemisch (G1) vollständig oder zumindest größtenteils entfernt.

Die im erfindungsgemäßen Verfahren in Schritt a) eingesetzte Destillationsvorrichtung (D1) kann prinzipiell jede dem Fachmann hierfür bekannte Destillationsvorrichtung, wie beispielsweise eine Kolonne, verwendet werden. Die konkrete Ausgestaltung der Destillationsvorrichtung (D1) wird im vorliegenden Text weiter unten verdeutlicht.

Die erfindungsgemäße Trennung des Wassers von N-Methylethylendiamin bzw. des Wassers von EDA gelingt, wenn man die Destillation bei einem Druck oberhalb von 4,8 bar durchführt. Unter diesen Bedingungen existiert kein Ethylendiamin/Wasser-Azeotop bzw. N-Methylethylendiamin/Wasser-Azeotrop.

Wie weiter unten noch genauer dargestellt, kann beispielsweise zunächst der Reaktionsaustrag aus der Hydrierung von Aminoacetonitril bzw. der Umsetzung von Ethanolamin mit Ammoniak in den Destillationsvorrichtungen (D1) und (D4) von Ammoniak befreit werden. Der Reaktionsaustrag der Destillationsvorrichtung (D4) kann somit zur Wasser-Abtrennung in die Destillationsvorrichtung (D1) zugeleitet werden.

Über den Kopf der Destillationsvorrichtung (D1) wird erfindungsgemäß das Wasser weitgehend oder vollständig abgetrennt. Im Rahmen der vorliegenden Erfindung wird unter dem Begriff "weitgehend frei von Wasser "bzw. "weitgehende Abtrennung des Wassers" folgendes verstanden : Die im Sumpfprodukt der Destillationsvorrichtung (D1) verbleibende Wassermenge beträgt weniger als 1000 Gew.-ppm, bevorzugt weniger als 200 Gew.-ppm, insbesondere weniger als 50 Gew.-ppm.

Als Sumpfprodukt der Destillationsvorrichtung (D1) fällt ein Reaktionsgemisch (G2) an, das im Wesentlichen Ethylendiamin, N-Methylethylendiamin und gegebenenfalls Diethylentriamin enthält.

Der Kopfdruck der Destillationsvorrichtung (D1) beträgt vorzugsweise mehr als 4,8 bis 20 bar, mehr bevorzugt 5 bis 10 bar, besonders bevorzugt 5 bis 6 bar.

Die Sumpftemperatur der Destillationsvorrichtung (D1) beträgt vorzugsweise 175 bis 250°C, mehr bevorzugt 175 bis 220°C, besonders bevorzugt 180 bis 200°C.

Das abgetrennte Wasser enthält vorzugsweise weniger als 1 Gew.-%, mehr bevorzugt weniger als 1000 Gew.-ppm, besonders bevorzugt weniger als 100 Gew.-ppm Stickstoff in Form von organischen Stickstoffverbindungen, insbesondere in Form von Aminen.

In einer bevorzugten Ausführungsform beträgt in Schritt a) die Sumpftemperatur in der Destillationsvorrichtung (D1) 175 bis 250 °C und/oder aus dem Gemisch (G1) wird das darin enthaltene Wasser vollständig oder zumindest weitgehend über Kopf aus (D1) abdestilliert. Weiterhin ist es in dieser Ausführungsform bevorzugt, dass in Schritt a) das aus dem Sumpf der Destillationsvorrichtung (D1) abgezogene Gemisch (G2) weniger als 1000 Gew.-ppm an Wasser enthält, mehr bevorzugt weniger als 200 Gew.-ppm Wasser, insbesondere weniger als 50 Gew.-ppm Wasser.

In Schritt b) erfolgt die Einspeisung des Gemisches (G2) in eine Destillationsvorrichtung (D2), wobei aus (D2) i) über Kopf ein Strom (S2) enthaltend N-MeEDA abdestilliert wird, und ii) aus dem Sumpf ein Strom (S3) abgezogen wird, wobei der Strom (S3) gegenüber dem Gemisch (G2) an N-MeEDA abgereichert ist.

Als Kopfprodukt der Destillationsvorrichtung (D2) wird N-Methylethylendiamin entnommen, das vorzugsweise 20 bis 50 Gew.-ppm., mehr bevorzugt 22 bis 40 Gew.-ppm, besonders bevorzugt 23 bis 30 Gew.-ppm Ethylendiamin enthält.

Über Sumpf der Destillationsvorrichtung (D2) geht ein Gemisch aus Ethylendiamin und gegebenenfalls Diethylentriamin, das vorzugsweise 0 bis 10.000 ppm, bevorzugt 0 bis 1.000 ppm, besonders bevorzugt 0 bis 200 ppm N-Methylethylendiamin enthält (Gew.-ppm).

Die Sumpftemperatur der Destillationsvorrichtung (D2) beträgt vorzugsweise 20 bis 75°C, mehr bevorzugt 40 bis 70°C, besonders bevorzugt 55 bis 65°C.

Der Kopfdruck der Destillationsvorrichtung (D2) liegt vorzugsweise bei 10 bis 500 mbar, mehr bevorzugt 30 bis 300 mbar, besonders bevorzugt 50 bis 200 mbar.

Die Destillationsvorrichtung (D2) enthält normalerweise 50 bis 200 theoretische Trennstufen, bevorzugt 70 bis 150 theoretische Trennstufen, besonders bevorzugt 80 bis 120 theoretische Trennstufen. Bevorzugt sind dabei druckverlustarme Packungen.

Das Verhältnis von Rücklaufmenge zu Zulaufmenge beträgt normalerweise 0,5 bis 100, bevorzugt 2,0 bis 10, besonders bevorzugt 3,0 bis 5.

Pro kg Ethylendiamin fallen vorzugsweise 0,02 bis 0,04 kg N-Methylethylendiamin an, die im erfindungsgemäßen Schritt b) abgetrennt werden.

In einer bevorzugten Ausführungsform der vorliegenden Erfindung wird in Schritt b) die Destillationsvorrichtung (D2) bei einer Sumpftemperatur von 20 bis 75 °C und/oder bei einem Kopfdruck von 10 bis 500 mbara, insbesondere von 50 bis 200 mbara, betrieben. Weiterhin ist es in dieser Ausführungsform bevorzugt, dass in Schritt b) der aus dem Sumpf der Destillationsvorrichtung (D2) abgezogene Strom (S3) weniger als 10000 Gew.-ppm an N-MeEDA enthält, mehr bevorzugt weniger als 1000 Gew.-ppm N-MeEDA, insbesondere weniger als 200 Gew.-ppm N-MeEDA. Ebenso ist es in dieser Ausführungsform bevorzugt, dass in Schritt b) der über Kopf aus der Destillationsvorrichtung (D2) abdestillierte Strom (S2) 20 bis 50 Gew.-ppm EDA enthält, mehr bevorzugt 22 bis 40 Gew.-ppm EDA, insbesondere 23 bis 30 Gew.-ppm EDA.

Sofern das Gemisch (G1) zusätzlich Ammoniak (NH₃) enthält, wird vorzugsweise vor Schritt a) in Schritt c) eine Ammoniakabtrennung aus dem Gemisch (G1) durchgeführt.

Vorzugsweise wird Schritt c) in zwei Stufen durchgeführt, wobei in zwei Stufen durchgeführt wird, wobei
in der ersten Stufe aus dem Gemisch (G1) in einer Destillationsvorrichtung (D3) Ammoniak über Kopf bei 20 bis 70 °C abgezogen und auskondensiert wird, die Sumpftemperatur kleiner als 220 °C ist und aus dem Sumpf ein an NH₃ abgereichertes Gemisch (G1a) in eine Destillationsvorrichtung (D4) überführt wird,
in der zweiten Stufe in der zweiten Destillationsvorrichtung (D4) das Gemisch (G1b) aus dem Sumpf abgetrennt wird, wobei das Gemisch (G1b) (weitgehend) frei von Ammoniak ist und das Gemisch (G1b) anstelle des Gemisches (G1) in Schritt a) in die Destillationsvorrichtung (D1) eingespeist wird.

Als Destillationsvorrichtungen (D3) und (D4) können alle dem Fachmann hierfür bekannten Destillationsvorrichtungen verwendet werden. In der Destillationsvorrichtung (D3) beträgt die Temperatur beim Kolonnenkopf ("Kondensationstemperatur") vorzugsweise 20 bis 70 °C, insbesondere 35 bis 60 °C. Vorzugsweise wird die Temperatur in der Destillationsvorrichtung (D3), insbesondere im Kopfbereich, über den Druck geregelt. Der Druck kann anhand von dem Fachmann bekannten Dampfdrucktabellen von Ammoniak bestimmt werden. Die Sumpftemperatur der Destillationsvorrichtung (D3) ist vorzugsweise < 200 °C, besonders bevorzugt < 190 °C. Die Sumpftemperatur wird vorzugsweise über den Ammoniakgehalt eingestellt, der über Sumpf aus der Destillationsvorrichtung (D3) abgezogen wird. In der Regel wird eine Sumpftemperatur von 140 °C nicht unterschritten.

In der zweiten Stufe wird der Kolonnendruck vorzugsweise so eingestellt, dass der Sumpfaustrag der Destillationsvorrichtung (D4) ammoniakfrei bzw. weitgehend frei von Ammoniak ist. Weitgehend frei von Ammoniak ist der Sumpfaustrag dann, wenn der Ammoniakgehalt < 1 Gew.-%, vorzugsweise < 0,5 Gew.-% ist, ammoniakfrei ist der Sumpfaustrag dann, wenn der Ammoniakgehalt < 0,1 Gew.-%, vorzugsweise 0,01 Gew.-% ist.

Die Sumpftemperaturen in der Destillationsvorrichtung (D4) entsprechen den Sumpftemperaturen, die vorstehend im Zusammenhang mit der Destillationsvorrichtung (D3) aufgeführt worden sind. Aus dem oberen Teil der Destillationsvorrichtung (D4), vorzugsweise über den Kolonnenkopf, wird Ammoniak abgetrennt. Vorzugsweise enthält dieser Strom neben Ammoniak auch Wasser. Dabei wird der Wassergehalt vorzugsweise so eingestellt, dass die Kopftemperatur der Destillationsvorrichtung (D4) bzw. die Kondensationstemperatur den vorstehend für die Destillationsvorrichtung (D3) angegebenen Temperaturbereichen am Kolonnenkopf entspricht. Vorzugsweise wird der NH₃-haltige Strom, der vorzugsweise aus dem Kolonnenkopf von (D4) abgezogen wird und der zusätzlich Wasser enthält, in die Destillationsvorrichtung (D3) zur ersten Stufe der Ammoniakabtrennung rückgeleitet. Besonders bevorzugt führt man den Kondensator in Form einer geschlossenen Kondensation aus. Dies kann durch Rückvermischung des Kondensates (Einführung eines Kreislaufes über den Kondensator) oder durch Kondensation im Gleichstrom ausgeführt werden.

Weiterhin ist es in dem erfindungsgemäßen Verfahren bevorzugt, dass im Gemisch (G1) enthaltene EDA durch ein Verfahren (A) oder ein Verfahren (B) hergestellt wird, wobei das Verfahren (A) die Schritte (A1) bis (A3) umfasst:
A1) Umsetzung von Formaldehyd und Blausäure (HCN) zu Formaldehydcyanhydrin (FACH), wobei die Blausäure vollständig frei oder weitgehend frei von Schwefeldioxid (SO₂) ist,
A2) Umsetzung von FACH mit Ammoniak (NH₃) zu Aminoacetonitril (AAN),
A3) Hydrierung von AAN in Gegenwart eines Katalysators unter Erhalt von EDA,
und das Verfahren (B) die Schritte (B1) und (B2) umfasst:
B1) Umsetzung von Ethylenoxid (EO) mit Ammoniak (NH₃) zu Ethanolamin (EOA),
B2) Umsetzung von EOA mit NH₃ zu EDA.

Die vorstehend beschriebenen Verfahren (A) und (B) als solche, sind dem Fachmann bekannt. Weiterhin ist es bevorzugt, dass das im erfindungsgemäßen Verfahren eingesetzte EDA, sofern es nach einem der beiden vorstehend beschriebenen Verfahren hergestellt wurde, direkt im Anschluss an die Hydrierung von AAN gemäß Schritt A3) oder an die Umsetzung von EOA gemäß Schritt B2) einer Ammoniakabtrennung unterzogen wird, vorzugsweise wie vorstehend beschrieben.

Weiterhin ist es bevorzugt, dass es im erfindungsgemäßen Verfahren das im Gemisch (G1) enthaltene EDA nach dem vorstehend beschriebenen Verfahren (A), umfassend die Schritte A1) bis A3), durchgeführt wird. Das Verfahren (A) wird im nachfolgenden Text näher präzisiert.

Im erfindungsgemäßen Verfahren erfolgt in Schritt A1) die Umsetzung von Formaldehyd und Blausäure (HCN) zu Formaldehydcyanhydrin (FACH), wobei die Blausäure vollständig frei oder weitgehend frei von Schwefeldioxid (SO₂) ist. Formaldehyd ist eine im Handel allgemein erhältliche Chemikalie. Vorzugsweise wird Formaldehyd in Form einer wässrigen Lösung eingesetzt. Bevorzugt handelt es sich dabei um wässrigen Formaldehyd mit einem Formaldehydanteil von 20 bis 60 Gew.-% [Mol/Mol], besonders bevorzugt mit einem Formaldehydanteil von 25 bis 55 Gew.-%.

Blausäure ist ebenfalls eine im Handel allgemein erhältliche Chemikalie. Blausäure kann großtechnisch nach im Wesentlichen drei verschiedenen Verfahren hergestellt werden. Nach einem ersten Verfahren kann Blausäure durch Ammoxidation von Methan mit Sauerstoff und Ammoniak (Andrussow-Verfahren) erhalten werden. Nach einem zweiten Verfahren kann Blausäure aus Methan und Ammoniak durch Ammondehydrierung in Abwesenheit von Sauerstoff erhalten werden. Schließlich kann Blausäure großtechnisch durch Dehydratisierung von Formamid hergestellt werden.

Blausäure kann flüssig oder gasförmig, in reiner Form oder als wässrige Lösung eingesetzt werden. Bevorzugt wird Blausäure als 50 bis 100 gew.-%ige, besonders bevorzugt als 75 bis 100 gew.-%ige wässrige Lösung eingesetzt. Blausäure wird vorzugsweise in einer Reinheit von 90 Gew.-% oder mehr eingesetzt.

Wie vorstehend bereits ausgeführt (inklusive der konkreten Zahlenwerte), kann im Rahmen des erfindungsgemäßen Verfahrens die Blausäure vollständig frei oder weitgehend frei von Schwefeldioxid (SO₂) sein. Schwefeldioxid kann als Stabilisator der Blausäure (beispielsweise nach deren Herstellung) direkt zugegeben werden. Dem Fachmann ist bekannt, dass sich in Gegenwart von Wasser mit SO₂ schwefelige Säure (H₂SO₃) bildet. Messmethoden zur Bestimmung des SO₂-Gehaltes von Blausäure sind dem Fachmann bekannt, beispielsweise kann dies durch Ionenchromatographie erfolgen.

Da in der kommerziell erhältlichen Blausäure in aller Regel Stabilisatoren, insbesondere saure Stabilisatoren, enthalten sind, wird im Rahmen der vorliegenden Erfindung entweder frisch synthetisierte und somit stabilisatorfreie Blausäure eingesetzt oder die vorhandenen Stabilisatoren, insbesondere Schwefeldioxid, werden nach dem Fachmann bekannten Methoden direkt vor dem Einsatz in Schritt A1) entfernt. Stabilisatorfreie Blausäure, ausgehend von nicht-flüchtige Stabilisatoren, insbesondere Schwefelsäure oder Phosphorsäure, enthaltender Blausäure, lässt sich nach den in WO 2004/092068 beschriebenen Methoden destillativ herstellen. Dabei wird die Blausäure über Kopf abdestilliert, die entsprechenden Stabilisatoren fallen als Sumpfprodukte an.

Stabilisatorfreie Blausäure, ausgehend von flüchtige Stabilisatoren, insbesondere von Schwefeldioxid, enthaltender Blausäure, kann gemäß US-A 2 571 099 infolge von Durchleiten eines Inertgases durch die Blausäure erhalten werden.

In einer bevorzugten Ausführungsform der vorliegenden Erfindung wird in Schritt A1) Blausäure eingesetzt, die vollständig frei oder weitgehend frei von Stabilisatoren ist. Die entsprechenden Zahlenwerte für die Begriffe "vollständig frei" bzw. "weitgehend frei" wurden vorstehend bereits definiert. Als Stabilisatoren werden in diesem Zusammenhang insbesondere Schwefeldioxid, Schwefelsäure, Phosphorsäure, Essigsäure, Oxalsäure sowie gegebenenfalls sonstige Säuren angesehen, die dem Fachmann als saure Stabilisatoren für Blausäure bekannt sind.

Die Umsetzung von Formaldehyd, vorzugsweise wässrigem Formaldehyd, und Blausäure zu FACH kann diskontinuierlich, halbkontinuierlich oder kontinuierlich durchgeführt werden. Vorzugsweise erfolgt sie in einem rückvermischten Reaktor mit Wärmeabfuhr, beispielsweise unter Verwendung eines Wärmetauschers. Als Reaktoren zur Durchführung von Schritt A1) können insbesondere Rührreaktoren, Schlaufenreaktoren oder Rohrreaktoren verwendet werden.

Schritt A1) kann prinzipiell bei beliebigen Temperaturen durchgeführt werden, vorzugsweise beträgt die Reaktionstemperatur 0 bis 70 °C, mehr bevorzugt 10 bis 50 °C, besonders bevorzugt 20 bis 45 °C.

Der Druck in Schritt A1) wird dabei so gewählt, dass das Reaktionsgemisch flüssig vorliegt.

Vorzugsweise wird die Blausäure gegenüber Formaldehyd äquimolar oder in einem leichten Unterschuss gefahren. Mehr bevorzugt beträgt das Molverhältnis von HCN zu Formaldehyd 0,85 bis 1,0 zu 1 [Mol/Mol], noch mehr bevorzugt 0,9 bis 1,0 zu 1 [Mol/Mol], insbesondere 0,95 bis 1,0 zu 1 [Mol/Mol].

Weiterhin ist es bevorzugt, dass das Reaktionsgemisch mit Hilfe einer Base, vorzugsweise mit Natronlauge, auf einen pH-Wert von 3,5 bis 6,5, bevorzugt 4,0 bis 6,0, besonders bevorzugt von 5,5 eingestellt wird.

Die Verweilzeit in der FACH-Synthese beträgt 1 Minute bis 1 Stunde, bevorzugt 5 Minuten bis 30 Minuten.

Der HCN-Umsatz in der FACH-Synthese beträgt > 99 % (bestimmt durch Titration nach Volhard), die FACH-Ausbeute beträgt > 98 % (bestimmt durch kombinierte Titration nach Volhard und Liebig).

Falls die erhaltene, vorzugsweise wässrige, FACH-Lösung beispielsweise auf eine 50 bis 80 gew.-%ige Lösung aufkonzentriert werden soll, ist es vorteilhaft, den pH-Wert im Reaktionsgemisch von Schritt A1) auf Werte < 5,5, bevorzugt < 3,5, abzusenken. Dies kann beispielsweise durch Zugabe von Mineralsäuren wie Schwefelsäure oder Phosphorsäure erreicht werden.

Im erfindungsgemäßen Verfahren erfolgt in Schritt A2) die Umsetzung von FACH mit Ammoniak (NH₃) zu Aminoacetonitril (AAN).

Erfindungsgemäß setzt man im Allgemeinen den Reaktionsaustrag aus Schritt A1), also das FACH bzw. ein FACH enthaltendes Gemisch, ohne Aufarbeitung und ohne die zusätzliche Verwendung eines Lösungsmittels mit Ammoniak zu AAN um. Ammoniak kann in Schritt A2) nicht nur als Edukt bei der AAN-Herstellung, sondern auch als Lösungsmittel verwendet werden. Gegebenenfalls kann eine in Schritt A1) erhaltene wässrige FACH-Lösung durch Abdampfen von Wasser aufkonzentriert werden.

Die Umsetzung von FACH mit Ammoniak kann diskontinuierlich, halbkontinuierlich oder kontinuierlich erfolgen. Die Umsetzung kann in jeder geeigneten und dem Fachmann bekannten Vorrichtung erfolgen. Vorzugsweise wird in Schritt A2) ein adiabates oder ein gekühltes Strömungsrohr ohne Rückvermischung oder ein Reaktor mit Kolbenströmungscharakteristik eingesetzt. Auf diese Weise kann die Bildung von störenden Nebenkomponenten aus FACH und AAN, erkennbar an der Farbe, vermieden werden.

In Schritt A2) beträgt die Temperatur in der Regel 0 bis 150 °C, bevorzugt 50 bis 100 °C, besonders bevorzugt 70 bis 90 °C.

In Schritt A2) kann der Druck prinzipiell beliebig eingestellt werden. Vorzugsweise beträgt in Schritt A2) der Druck 20 bis 400 bar, insbesondere 80 bis 270 bar. Vorzugsweise ist in Schritt A2) der Druck so hoch ist, dass das Reaktionsgemisch flüssig ist. Weiterhin vorzugsweise ist der Druck in Schritt A2) höher als in Schritt A3). Beispielsweise kann der Druck in Schritt A2) um 5 bis 20 bar höher sein als in Schritt A3).

Das Molverhältnis zwischen FACH und Ammoniak kann prinzipiell beliebig sein, in der Regel wird jedoch zumindest eine äquimolare Menge an Ammoniak eingesetzt, vorzugsweise wird Ammoniak in hohem molarem Überschuss zu FACH gefahren. Vorzugsweise beträgt in Schritt A2) das Molverhältnis von FACH zu Ammoniak 1 : 2 bis 1 : 15 [Mol/Mol], mehr bevorzugt 1 : 5 bis 1 : 30 [Mol/Mol], insbesondere 1 : 10 bis 1 : 20 [Mol/Mol].

Die Verweilzeit des Reaktionsgemisches in der entsprechenden Vorrichtung beträgt vorzugsweise 0,1 bis 20 Minuten, besonders bevorzugt 1,0 bis 10 Minuten.

Die AAN-Ausbeute beträgt (bezogen auf FACH) vorzugsweise ≥ 95 %. Weiterhin ist das Gewichtsverhältnis von AAN zu IDAN vorzugsweise ≥ 99 : 1.

In Schritt A2) enthält der Reaktionsaustrag vorzugsweise 10 bis 50 Gew.-% AAN, 10 bis 80 Gew.-% Ammoniak, < 1 Gew.-% FACH, < 1 Gew.-% IDAN. Der Rest ist Wasser, das in Schritt A2) bei der Herstellung von AAN gebildet wird bzw. bereits bei der FACH-Herstellung, gemeinsam mit den Edukten, eingesetzt wurde.

In einer bevorzugten Ausführungsform der vorliegenden Erfindung wird das in Schritt A2) erhaltene AAN
i) ohne destillative Aufarbeitung und/oder ohne Wasserabtrennung als Roh-AAN in Schritt A3) hydriert, oder
ii) einer adsorptiven Reinigung unter Verwendung eines Ionentauschers oder eines Metalloxids unterzogen, bevor es gemäß Schritt A3) hydriert wird.

Im erfindungsgemäßen Verfahren wird in Schritt A3) die Hydrierung von AAN in Gegenwart eines Katalysators unter Erhalt von Ethylendiamin (EDA) durchgeführt.

Die Hydrierung unter Erhalt von EDA erfolgt im Allgemeinen durch Umsetzung von AAN mit Wasserstoff in Gegenwart des Katalysators ("Hydrierkatalysator"). Dabei werden mindestens zwei Mol Wasserstoff pro Mol AAN benötigt. Das in Schritt A2) erhaltene AAN kann direkt der Hydrierung gemäß Schritt A3) unterzogen werden, gegebenenfalls können aber noch Aufarbeitungsschritte zwischen Schritt A2) und Schritt A3) durchgeführt werden, wie weiter unten näher erläutert.

Der Wasserstoff kommt im Allgemeinen technisch rein zum Einsatz. Der Wasserstoff kann auch in Form eines Wasserstoff enthaltenden Gases, das heißt mit Beimengungen anderer Inertgase, wie Stickstoff, Helium, Neon, Argon oder Kohlendioxid, zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO, enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Als Katalysatoren können prinzipiell alle dem Fachmann für eine Nitrilhydrierung bekannten Katalysatoren eingesetzt werden. Als Katalysatoren zur Hydrierung (Hydrierkatalysatoren) der Nitrilfunktion von AAN können somit beispielsweise Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten.

Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten.

In einer besonders bevorzugten Ausführungsform werden bei Hydrierung von AAN Raney-Katalysatoren eingesetzt, bevorzugt Raney-Kobalt- oder Raney-NickelKatalysatoren und besonders bevorzugt ein Raney-Kobalt-Katalysator, der als Promotor mindestens eines der Elemente Ni, Cr oder Fe enthält. Der Raney-Kobalt-Katalysator ist also mit mindestens einem dieser Elemente dotiert. Erfindungsgemäß werden die Raney-Katalysatoren bevorzugt als suspendierte Raney-Katalysatoren eingesetzt.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

In einer bevorzugten Form der vorliegenden Erfindung wird im optionalen Schritt c) NH₃ abgetrennt und in einen vorausgehenden Verfahrensschritt rückgeführt, vorzugsweise erfolgt die Ammoniakrückführung nach Schritt A2) von Verfahren (A) oder nach mindestens einem der beiden Schritte B1) oder B2) von Verfahren (B).

Weiterhin ist es im erfindungsgemäßen Verfahren bevorzugt, dass das Verfahren einen zusätzlichen Schritt d) umfasst:
d) Einspeisung des Stroms (S3) in eine Destillationsvorrichtung (D5), wobei aus D5 über Kopf bei einem Druck von 200 mbara bis 2 bara EDA abdestilliert wird, vorzugsweise hat das EDA eine Reinheit von mindestens 95 %, mehr bevorzugt von mindestens 99 %, insbesondere von mindestens 99,5 %.

Die Sumpftemperatur der Destillationsvorrichtung (D5) beträgt normalerweise 150 bis 250°C, bevorzugt 170 bis 220°C, besonders bevorzugt 175 bis 185°C.

Der Kopfdruck der Destillationsvorrichtung (D5) beträgt normalerweise 200 mbar bis 2 bar, bevorzugt 250 mbar bis 500 mbar, besonders bevorzugt 250 mbar bis 350 mbar.

Sofern im erfindungsgemäßen Verfahren im Gemisch (G1) zusätzlich Diethylentriamin (DETA) enthalten ist, wird erfindungsgemäß eine DETA-Abtrennung als zusätzlicher Schritt e) durchgeführt, vorzugsweise erfolgt der Schritt e) im Anschluss an Schritt b) und/oder Schritt d), besonders bevorzugt ist die Schrittreihenfolge b), d), e).

Weiterhin ist es bevorzugt, dass DETA im Strom (S3) enthalten ist und aus der Destillationsvorrichtung (D5) aus dem Sumpf ein an DETA angereicherter Strom (S4) abgezogen wird und der Schritt e) wie folgt durchgeführt wird:
e) Einspeisung des Stroms (S4) in eine Destillationsvorrichtung (D6), wobei aus D6 über einen Seitenabzug DETA abdestilliert wird.

Die Sumpftemperatur der Destillationsvorrichtung (D6) beträgt normalerweise 100 bis 250°C, bevorzugt 170 bis 220°C, besonders bevorzugt 175°C bis 200°C.

Der Kopfdruck der Destillationsvorrichtung (D6) beträgt normalerweise 10 bis 500 mbar, bevorzugt 20 bis 100 mbar, besonders bevorzugt 30 bis 60 mbar.

Gegebenenfalls können aus der Destillationsvorrichtung (D6) zusätzlich als Kopfprodukt verbliebene Leichtsieder und/oder als Sumpfprodukt verbliebe Hochsieder abgetrennt werden.

Die im erfindungsgemäßen Verfahren durchgeführten Destillationen unter Verwendung der vorstehend beschriebenen Destillationsvorrichtungen (D1) bis (D6) können in jeder geeigneten, dem Fachmann bekannten Vorrichtung durchgeführt werden. Für diese Destillationen geeignet sind Apparaturen, wie sie beispielsweise in: Kirk-Othmer, Encyclopedia of Chemical Technology, 4. Aufl., Vol.8, John Wiley & Sons, New York, 1996, Seiten 334 bis 348, beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer.

Bevorzugt weisen die Destillationskolonnen (D1) bis (D6) Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in ein oder mehreren Betten vorliegen.

Die Zahl der theoretischen Böden in der Destillationsvorrichtung (D1) beträgt 50 bis 100, bevorzugt 55 bis 70, besonders bevorzugt 60 bis 65, in der Destillationsvorrichtung (D2) 70 bis 150, bevorzugt 80 bis 120, besonders bevorzugt 90 bis 110, in der Destillationsvorrichtung (D5) 15 bis 50, bevorzugt 20 bis 40, besonders bevorzugt 23 bis 30, in der Destillationsvorrichtung (D6) 5 bis 30, bevorzugt 7 bis 20, besonders bevorzugt 8 bis 15.

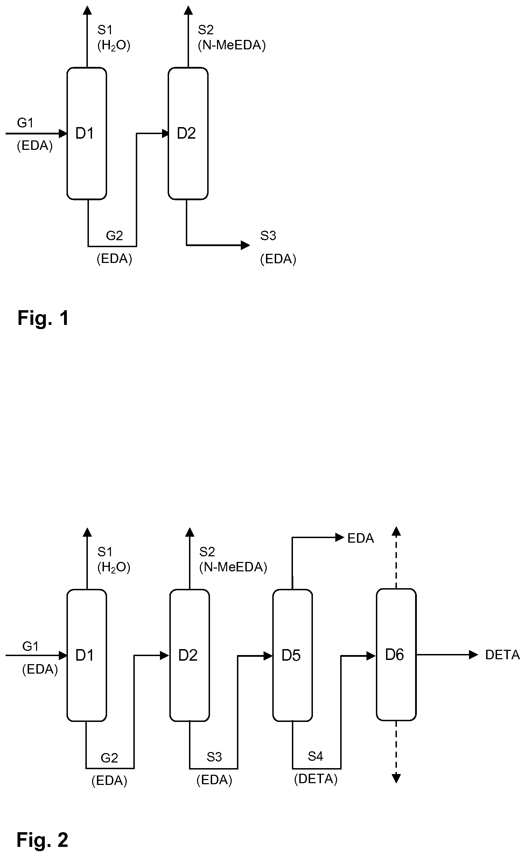

In Figur 1 wird das erfindungsgemäße Verfahren in seiner Grundform nochmals verdeutlicht. In Klammern sind unter den jeweiligen Gemischen oder Strömen die wichtigsten darin enthaltenen Komponenten aufgeführt.

Eine bevorzugte Ausführungsform der vorliegenden Erfindung wird in Figur 2 zusätzlich verdeutlicht. In Figur 2 haben die Abkürzungen, Pfeile und sonstige Symbole eine sinngemäße Bedeutung, wie vorstehend für Figur 1, ausgeführt. Gegenüber der Ausführungsform gemäß Figur 1 wird in der Ausführungsform gemäß Figur 2, zusätzlich eine EDA-Abtrennung über die Destillationsvorrichtung (D5) sowie eine DETA-Abtrennung über die Destillationsvorrichtung (D6) durchgeführt. DETA wird dabei über einen Seitenabzug von (D6) gewonnen. Die gestrichelten Linien bedeuten, dass gegebenenfalls weitere Komponenten wie Strom (S4) enthalten sein können, die über den Kopf und/oder den Sumpf von (D6) abgetrennt werden können.

Eine weitere bevorzugte Ausführungsform der vorliegenden Erfindung wird in Figur 3 zusätzlich verdeutlicht, wobei auf die vorstehenden Ausführungen zu den Figuren 1 und 2 Bezug genommen wird. Bei dieser Ausführungsform wird vor Durchführung des erfindungsgemäßen Schrittes a) eine Ammoniaktrennung gemäß Schritt c) durchgeführt. Wie aus Figur 3 ersichtlich, ist die Ammoniakabtrennung zweistufig unter Verwendung der Destillationsvorrichtungen (D3) und (D4). Bei dieser Ausführungsform ist im eingesetzten Gemisch (G1) also zusätzlich Ammoniak enthalten. Folglich wird in die Destillationsvorrichtung (D1) gemäß Schritt a) des erfindungsgemäßen Verfahrens anstelle des Gemisches (G1) das Gemisch (G1d) eingespeist.

Eine weitere bevorzugte Ausführungsform der vorliegenden Erfindung wird in Figur 4 zusätzlich verdeutlicht. Bei dieser Ausführungsform wird das im Gemisch (G1) enthaltene EDA wie in dem vorstehend beschriebenen Verfahren (A) hergestellt und zusätzlich im Anschluss an die Hydrierung gemäß Verfahrensschritt (A3) eine zweistufige Ammoniakabtrennung aus dem Gemisch (G1) durchgeführt, analog zu der vorstehend beschrieben Ausführungsform gemäß Figur 3. "FA" bedeutet Formaldehyd, "B" bedeutet Base. Die Verwendung einer Base ist hier nur optional, was durch die gestrichelte Linie angedeutet ist. Die Verfahrensschritte (A1) bis (A3) werden vorzugsweise in den entsprechenden Reaktoren durchgeführt, die in Figur 4 sinngemäß als "R1" bis "R3" dargestellt sind. Die Hauptkomponenten (Edukte oder Produkte) der Schritte (A1) bis (A3) sind unter Verwendung von Pfeilen entsprechend aufgeführt, in den Klammern sind die wichtigsten Nebenprodukte beziehungsweise nicht umgesetzten Edukte der einzelnen Schritte angegeben.

In Figur 4 ist somit nur ein Teilbereich dieser Ausführungsform des erfindungsgemäßen Verfahrens dargestellt, das aus der Destillationsvorrichtung (D4) erhaltene Gemisch (G1b) wird anschließend zumindest den erfindungsgemäßen Schritten a) und b) unterzogen, wie vorstehend in Figur 1 bildhaft dargestellt. Ebenfalls können auch die in Ausführungsform gemäß Figur 2 zusätzlich enthalten Verfahrensschritte zusammen mit den Destillationsvorrichtungen (D5) und (D6) durchgeführt werden. Dies ist insbesondere dann der Fall, wenn im Gemisch (G1) nennenswerte Mengen an DETA enthalten sind. Besonders bevorzugt wird die in Figur 4 bildhaft dargestellte Ausführungsform als Kombination mit der Ausführungsform gemäß Figur 2 durchgeführt.

Nachfolgend wird die vorliegende Erfindung anhand der Beispiele verdeutlicht.

### Beispiel 1: Ammoniakvorabtrennung

45.0 kg/h eines Stromes (Gemisch G1) enthaltend 64,1 % NH₃, 21,4% H₂O, 13,3% EDA, 0,35 % N-MeEDA, 0,46 % DETA und 0,39 % unbekannter Nebenkomponenten werden einer Abtriebskolonne D3 mit 3 theoretischen Trennstufen, die bei 18,0 bar_{abs} betrieben werden, zugeführt. Am Sumpf von D3 wird eine Temperatur von 180 °C eingestellt. In den Sumpf von D3 wird der Kopfaustrag aus der nachfolgenden Kolonne D4 gefahren. Dieser besteht aus 3,17 kg/h enthaltend 31,2 % NH₃, 58,9 % H₂O, 9,1 % EDA, 0,56 % N-MeEDA, 430 ppm DETA, Rest andere.

In D3 werden über Kopf 28,9 kg/h NH₃ mit 0,2 % H₂O abgezogen. Über Sumpf werden 19,29 kg/h mit 5,6 % NH3, 59,3% H₂O, 32,3 % EDA, 0,905% N-MeEDA, 1,08% DETA und 0,87 % andere abgezogen. Die Sumpftemperatur beträgt 180 °C.

Der Sumpfaustrag von D3 wird in eine Kolonne D4 mit einem Abtriebsteil mit 13 theoretischen Trennstufen geleitet. Die D4 wird bei einem Druck von 9,0 bar_{abs} betrieben. Über Kopf wird ein Gemisch abgezogen, welches durch Umlauf an einem Wärmetauscher bei 69 °C kondensiert wird. Der Kopfaustrag wird in die Kolonne D3 wie oben beschrieben zurückgeführt. Die Sumpftemperatur von D4 beträgt 183 °C. Über Sumpf wird ein Strom (Gemisch G1b) ausgetragen, der weniger als 10 ppm NH₃ enthält, mit 59,7% H₂O, 37,0 % EDA, 0,98% N-MeEDA, 1,30 % DETA und 1,0% andere.

### Beispiel 2:

Der Sumpfaustrag aus D4 gemäß Beispiel 1 wird zur Entwässerung in eine Kolonne D1 mit 60 theoretischen Trennstufen, 32 im Abtriebsteil und 28 im Verstärkungsteil, geleitet. D1 wird bei einem Druck von 5,4 barabs betrieben, die Kopftemperatur beträgt 154,8 °C, die Sumpftemperatur 182,5 °C. Über Kopf werden 9,57 kg/h Wasser mit 100 ppm EDA (N-MeEDA unter der Nachweisgrenze) gefahren. Über Sumpf werden 6,46 kg/h mit 92 ppm H₂O, 91,8% EDA, 2,4% N-MeEDA, 3,2 % DETA und 2,5% andere Nebenkomponenten gefahren und als Gemisch (G2) abgezogen.

Der Sumpfaustrag aus D1 wird in eine Kolonne D2 zur Abtrennung von N-MeEDA gefahren. Die Kolonne D2 enthält Packung mit einer theoretischen Trennnstufenzahl von 100, davon 46 im Abtriebsteil und 54 im Verstärkungsteil. D2 wird bei einem Kopfdruck von 100mbar betrieben. Der Druck im Sumpf beträgt 115mbar. Die Kopftemperatur beträgt 56,2 C, die Sumpftemperatur 60,4 C. Die Rücklaufmenge beträgt 27,3 kg/h. Über Kopf werden 0.2 kg/h gefahren mit 0,3 % H₂O, 24,9 % EDA und 74,8 % N-MeEDA. Über Sumpf werden 6,26 kg/h gefahren mit 94,0 % EDA, 0,1 % N-MeEDA, 3,3 % DETA und 2,6 % anderen Nebenkomponenten.

Wie aus Beispiel 2 ersichtlich, können mit dem erfindungsgemäßen Verfahren sowohl H₂O als auch N-MeEDA nahezu vollständig von EDA abgetrennt werden, ohne dass dabei eine azeotrope Destillation durchgeführt werden muss.

## Patentansprüche

1. Verfahren zur destillativen Reinigung von Ethylendiamin (EDA), umfassend die Schritte a) und b) mit:
a) Einspeisung eines Gemisches (G1) enthaltend Wasser, EDA und N-Methylethylendiamin (N-MeEDA) in eine Destillationsvorrichung (D1), wobei aus (D1)
i) über Kopf bei einem Druck von größer als 4,8 bara ein Strom (S1) enthaltend Wasser abdestilliert wird, und
ii) aus dem Sumpf ein Gemisch (G2) abgezogen wird, wobei das Gemisch (G2) gegenüber dem Gemisch (G1) an Wasser abgereichert ist,
b) Einspeisung des Gemisches (G2) in eine Destillationsvorrichtung (D2), wobei aus (D2)
i) über Kopf ein Strom (S2) enthaltend N-MeEDA abdestilliert wird, und
ii) aus dem Sumpf ein Strom (S3) abgezogen wird, wobei der Strom (S3) gegenüber dem Gemisch (G2) an N-MeEDA abgereichert ist.

2. Verfahren gemäß Anspruch 1, **dadurch gekennzeichnet, dass** das Gemisch (G1) zusätzlich Ammoniak (NH₃) enthält und vor Schritt a) als Schritt c) eine Ammoniakabtrennung aus dem Gemisch (G1) durchgeführt wird.

3. Verfahren gemäß Anspruch 2, **dadurch gekennzeichnet, dass** Schritt c) in zwei Stufen durchgeführt wird, wobei
in der ersten Stufe aus dem Gemisch (G1) in einer Destillationsvorrichtung (D3) Ammoniak über Kopf bei 20 bis 70 °C abgezogen und auskondensiert wird, die Sumpftemperatur kleiner als 220 °C ist und aus dem Sumpf ein an NH₃ abgereichertes Gemisch (G1a) in eine Destillationsvorrichtung (D4) überführt wird,
in der zweiten Stufe in der zweiten Destillationsvorrichtung (D4) das Gemisch (G1b) aus dem Sumpf abgetrennt wird, wobei das Gemisch (G1b) (weitgehend) frei von Ammoniak ist und das Gemisch (G1b) anstelle des Gemisches (G1) in Schritt a) in die Destillationsvorrichtung (D1) eingespeist wird.

4. Verfahren gemäß einem der Ansprüche 1 bis 3, **dadurch gekennzeichnet, dass** das im Gemisch (G1) enthaltene EDA durch ein Verfahren (A) oder ein Verfahren (B) hergestellt wird, wobei
i) das Verfahren (A) die Schritte (A1) bis (A3) umfasst:
A1) Umsetzung von Formaldehyd und Blausäure (HCN) zu Formaldehydcyanhydrin (FACH), wobei die Blausäure vollständig frei oder weitgehend frei von Schwefeldioxid (SO₂) ist,
A2) Umsetzung von FACH mit Ammoniak (NH₃) zu Aminoacetonitril (AAN),
A3) Hydrierung von AAN in Gegenwart eines Katalysators unter Erhalt von EDA,
ii) das Verfahren (B) die Schritte (B1) und (B2) umfasst:
B1) Umsetzung von Ethylenoxid (EO) mit Ammoniak (NH₃) zu Ethanolamin (EOA),
B2) Umsetzung von EOA mit NH₃ zu EDA.

5. Verfahren gemäß Anspruch 4, **dadurch gekennzeichnet, dass** im Verfahren (A) in Schritt A3) ein Raney-Katalysator eingesetzt wird, bevorzugt ein Raney-Nickel- oder ein Raney-Kobalt-Katalysator, insbesondere ein Raney-Kobalt-Katalysator, der als Promotor mindestens eines der Elemente Fe, Ni oder Cr enthält.

6. Verfahren gemäß einem der Ansprüche 1 bis 5, **dadurch gekennzeichnet, dass** das Verfahren einen zusätzlichen Schritt d) umfasst mit:
d) Einspeisung des Stroms (S3) in eine Destillationsvorrichtung (D5), wobei aus D5 über Kopf bei einem Druck von 200 mbara bis 2 bara EDA abdestilliert wird, vorzugsweise hat das EDA eine Reinheit von mindestens 95 %, mehr bevorzugt von mindestens 99 %, insbesondere von mindestens 99,5%.

7. Verfahren gemäß einem der Ansprüche 1 bis 6, **dadurch gekennzeichnet, dass** das Gemisch (G1) zusätzlich Diethylentriamin (DETA) enthält und eine DETA-Abtrennung als zusätzlicher Schritt e) durchgeführt wird, vorzugsweise erfolgt der Schritt e) im Anschluss an Schritt b) und/oder Schritt d), besonders bevorzugt ist die Schrittreihenfolge b), d), e).

8. Verfahren gemäß Anspruch 7, **dadurch gekennzeichnet, dass** DETA im Strom (S3) enthalten ist und aus der Destillationsvorrichtung (D5) aus dem Sumpf ein an DETA angereicherter Strom (S4) abgezogen wird und der Schritt e) wie folgt durchgeführt wird:
e) Einspeisung des Stroms (S4) in eine Destillationsvorrichtung (D6), wobei aus D6 über einen Seitenabzug DETA abdestilliert wird.

9. Verfahren gemäß einem der Ansprüche 1 bis 8, **dadurch gekennzeichnet, dass** in Schritt a) die Sumpftemperatur in der Destillationsvorrichtung (D1) 175 bis 250 °C beträgt und/oder aus dem Gemisch (G1) das darin enthaltene Wasser vollständig oder zumindest weitgehend über Kopf aus (D1) abdestilliert wird.

10. Verfahren gemäß einem der Ansprüche 1 bis 9, **dadurch gekennzeichnet, dass** in Schritt a) das aus dem Sumpf der Destillationsvorrichtung (D1) abgezogene Gemisch (G2) weniger als 1000 Gew.-ppm an Wasser enthält, mehr bevorzugt weniger als 200 Gew.-ppm Wasser, insbesondere weniger als 50 Gew.-ppm Wasser.

11. Verfahren gemäß einem der Ansprüche 1 bis 10, **dadurch gekennzeichnet, dass** in Schritt b) die Destillationsvorrichtung (D2) bei einer Sumpftemperatur von 20 bis 75 °C und/oder bei einem Kopfdruck von 10 bis 500 mbara, insbesondere von 50 bis 200 mbara, betrieben wird.

12. Verfahren gemäß einem der Ansprüche 1 bis 11, **dadurch gekennzeichnet, dass** in Schritt b) der aus dem Sumpf der Destillationsvorrichtung (D2) abgezogene Strom (S3) weniger als 10000 Gew.-ppm an N-MeEDA enthält, mehr bevorzugt weniger als 1000 Gew.-ppm N-MeEDA, insbesondere weniger als 200 Gew.-ppm N-MeEDA.

13. Verfahren gemäß einem der Ansprüche 1 bis 12, **dadurch gekennzeichnet, dass** in Schritt b) der über Kopf aus der Destillationsvorrichtung (D2) abdestillierte Strom (S2) 20 bis 50 Gew.-ppm EDA enthält, mehr bevorzugt 22 bis 40 Gew.-ppm EDA, insbesondere 23 bis 30 Gew.-ppm EDA.

14. Verfahren gemäß einem der Ansprüche 1 bis 13, **dadurch gekennzeichnet, dass** das Gemisch (G1) im Wesentlichen, vorzugsweise zu mindestens 99 Gew.-%, insbesondere zu mindestens 99,5 Gew.-%, EDA, N-MeEDA, DETA, Wasser und NH₃ enthält.

15. Verfahren gemäß einem der Ansprüche 1 bis 14, **dadurch gekennzeichnet, dass** in Schritt c) NH₃ abgetrennt und in einen vorausgehenden Verfahrensschritt rückgeführt wird, vorzugsweise erfolgt die Ammoniakrückführung nach Schritt A2) von Verfahren (A) oder nach mindestens einem der beiden Schritte B1) oder B2) von Verfahren (B).

## Claims

1. A process for purifying ethylenediamine (EDA) by distillation, which comprises the step a) and b), namely:
a) introduction of a mixture (G1) comprising water, EDA and N-methylethylenediamine (N-MeEDA) into a distillation apparatus (D1), with
i) a stream (S1) comprising water being distilled off overhead from (D1) at a pressure of greater than 4.8 bara and
ii) a mixture (G2) depleted in water compared to the mixture (G1) being taken off from the bottom of (D1),
b) introduction of the mixture (G2) into a distillation apparatus (D2), with
i) a stream (S2) comprising N-MeEDA being distilled off overhead from (D2) and
ii) a stream (S3) depleted in N-MeEDA compared to the mixture (G2) being taken off from the bottom of (D2).

2. The process according to claim 1, wherein the mixture (G1) additionally comprises ammonia (NH₃) and a removal of ammonia from the mixture (G1) is carried out as step c) before step a).

3. The process according to claim 2, wherein step c) is carried out in two stages, where
in the first stage, ammonia is taken off overhead at from 20 to 70°C from the mixture (G1) in a distillation apparatus (D3) and is condensed, the temperature at the bottom is less than 220°C and a mixture (G1a) depleted in NH₃ is transferred from the bottom to a distillation apparatus (D4),
in the second stage, the mixture (G1b) is separated off from the bottom in the second distillation apparatus (D4), with the mixture (G1b) being (largely) free of ammonia and the mixture (G1b) being fed instead of the mixture (G1) into the distillation apparatus (D1) in step a).

4. The process according to any of claims 1 to 3, wherein the EDA comprised in the mixture (G1) is prepared by a process (A) or a process (B), where
i) the process (A) comprises the steps (A1) to (A3) :
A1) reaction of formaldehyde and hydrocyanic acid (HCN) to form formaldehyde cyanohydrin (FACH), where the hydrocyanic acid is completely or largely free of sulfur dioxide (SO₂),
A2) reaction of FACH with ammonia (NH₃) to form aminoacetonitrile (AAN),
A3) hydrogenation of AAN in the presence of a catalyst to give EDA,
ii) the process (B) comprises the steps (B1) and (B2) :
B1) reaction of ethylene oxide (EO) with ammonia (NH₃) to form ethanolamine (EOA),
B2) reaction of EOA with NH₃ to form EDA.

5. The process according to claim 4, wherein step (A3) of the process (A) is carried out using a Raney catalyst, preferably a Raney nickel catalyst or a Raney cobalt catalyst, in particular a Raney cobalt catalyst comprising at least one of the elements Fe, Ni or Cr as promoter.

6. The process according to any of claims 1 to 5, wherein the process comprises an additional step d):
d) introduction of the stream (S3) into a distillation apparatus (D5), with EDA being distilled off overhead from D5 at a pressure of from 200 mbara to 2 bara and the EDA preferably having a purity of at least 95%, more preferably at least 99%, in particular at least 99.5%.

7. The process according to any of claims 1 to 6, wherein the mixture (G1) additionally comprises diethylenetriamine (DETA) and a DETA removal is carried out as additional step e), with the step e) preferably being carried out after step b) and/or step d) and particular preference being given to the step sequence b), d), e).

8. The process according to claim 7, wherein DETA is comprised in the stream (S3) and a stream (S4) enriched in DETA is taken off from the bottom of the distillation apparatus (D5) and step e) is carried out as follows:
e) introduction of the stream (S4) into a distillation apparatus (D6), with DETA being distilled off via a side offtake from D6.

9. The process according to any of claims 1 to 8, wherein, in step a), the temperature at the bottom of the distillation apparatus (D1) is from 175 to 250°C and/or the water comprised in the mixture (G1) is completely or at least largely distilled off overhead from (D1).

10. The process according to any of claims 1 to 9, wherein the mixture (G2) taken off from the bottom of the distillation apparatus (D1) in step a) comprises less than 1000 ppm by weight of water, more preferably less than 200 ppm by weight of water, in particular less than 50 ppm by weight of water.

11. The process according to any of claims 1 to 10, wherein the distillation apparatus (D2) in step b) is operated at a temperature at the bottom of from 20 to 75°C and/or at a pressure at the top of from 10 to 500 mbara, in particular from 50 to 200 mbara.

12. The process according to any of claims 1 to 11, wherein the stream (S3) taken off from the bottom of the distillation apparatus (D2) in step b) comprises less than 10 000 ppm by weight of N-MeEDA, more preferably less than 1000 ppm by weight of N-MeEDA, in particular less than 200 ppm by weight of N-MeEDA.

13. The process according to any of claims 1 to 12, wherein the stream (S2) distilled off overhead from the distillation apparatus (D2) in step b) comprises from 20 to 50 ppm by weight of EDA, more preferably from 22 to 40 ppm by weight of EDA, in particular from 23 to 30 ppm by weight of EDA.

14. The process according to any of claims 1 to 13, wherein the mixture (G1) comprises essentially, preferably to an extent of at least 99% by weight, in particular at least 99.5% by weight, EDA, N-MeEDA, DETA, water and NH₃.

15. The process according to any of claims 1 to 14, wherein NH₃ is separated off in step c) and recirculated to a preceding process step, with the ammonia recirculation preferably being carried out after step A2) of process (A) or after at least one of the two steps B1) or B2) of process (B).

## Revendications

1. Procédé pour la purification par distillation d'éthylènediamine (EDA), comprenant les étapes a) et b) :
a) injection d'un mélange (G1) contenant de l'eau, de l'EDA et de la N-méthyléthylènediamine (N-MeEDA) dans un dispositif de distillation (D1), dans lequel, à partir de (D1)
i) on élimine par distillation via la tête, à une pression supérieure à 4,8 bara, un flux (S1) contenant de l'eau et
ii) on soutire du fond un mélange (G2), le mélange (G2) étant appauvri en eau par rapport au mélange (G1),
b) injection du mélange (G2) dans un dispositif de distillation (D2), dans lequel, à partir de (D2)
i) on élimine par distillation via la tête un flux (S2) contenant de la N-MeEDA et
ii) on soutire du fond un flux (G3), le flux (G3) étant appauvri en N-MeEDA par rapport au mélange (G2).

2. Procédé selon la revendication 1, **caractérisé en ce que** le mélange (G1) contient en outre de l'ammoniac (NH₃) et une séparation de l'ammoniac du mélange (G1) est réalisée en tant qu'étape c) avant l'étape a).

3. Procédé selon la revendication 2, **caractérisé en ce que** l'étape c) est réalisée en deux étapes, où dans la première étape, de l'ammoniac est soutiré à partir du mélange (G1) dans un dispositif de distillation (D3) via la tête à 20 jusqu'à 70°C et est séparé par condensation, la température du fond est inférieure à 220°C et un mélange (G1a) appauvri en NH3 est transféré du fond dans un dispositif de distillation (D4),
dans la deuxième étape, dans le deuxième dispositif de distillation (D4), le mélange (G1b) est séparé du fond, le mélange (G1b) étant (dans une large mesure) exempt d'ammoniac et le mélange (G1b) étant injecté dans le dispositif de distillation (D1) à la place du mélange (G1) dans l'étape a).

4. Procédé selon l'une quelconque des revendications 1 à 3, **caractérisé en ce que** l'EDA contenue dans le mélange (G1) est préparée par un procédé (A) ou par un procédé (B)
i) le procédé (A) comprenant les étapes (A1) à (A3) :
A1) transformation de formaldéhyde et d'acide cyanhydrique (HCN) en cyanhydrine de formaldéhyde (FACH), l'acide cyanhydrique étant totalement ou dans une large mesure exempt de dioxyde de soufre (SO₂),
A2) transformation de FACH avec de l'ammoniac (NH₃) en aminoacétonitrile (AAN),
A3) hydrogénation d'AAN en présence d'un catalyseur avec obtention d'EDA,
ii) le procédé (B) comprenant les étapes (B1) et (B2) :
B1) transformation d'oxyde d'éthylène (OE) avec de l'ammoniac (NH₃) en éthanolamine (EOA),
B2) transformation d'EOA avec du NH₃ en EDA.

5. Procédé selon la revendication 4, **caractérisé en ce qu'**on utilise, dans le procédé (A), dans l'étape A3), un catalyseur de Raney, de préférence un catalyseur à base de nickel de Raney ou de cobalt de Raney, en particulier un catalyseur à base de cobalt de Raney, qui contient comme promoteur au moins un des éléments Fe, Ni ou Cr.

6. Procédé selon l'une quelconque des revendications 1 à 5, **caractérisé en ce que** le procédé comprend une étape d) supplémentaire :
d) injection du flux (S3) dans un dispositif de distillation (D5), de l'EDA étant éliminée par distillation à partir de (D5) via la tête à une pression de 200 mbara à 2 bara, l'EDA présentant de préférence une pureté d'au moins 95%, plus préférablement d'au moins 99%, en particulier d'au moins 99,5%.

7. Procédé selon l'une quelconque des revendications 1 à 6, **caractérisé en ce que** le mélange (G1) contient en outre de la diéthylènetriamine (DETA) et une séparation de DETA est réalisée en tant qu'étape supplémentaire e), l'étape e) ayant de préférence lieu consécutivement à l'étape b) et/ou à l'étape d), l'ordre des étapes étant de manière particulièrement préférée b), d), e).

8. Procédé selon la revendication 7, **caractérisé en ce que** la DETA est contenue dans le flux (S3) et on soutire du fond du dispositif de distillation (D5) un flux (S4) enrichi en DETA et l'étape e) est réalisée comme suit :
e) injection du flux (S4) dans un dispositif de distillation (D6), de la DETA étant éliminée par distillation via un soutirage latéral à partir de (D6) .

9. Procédé selon l'une quelconque des revendications 1 à 8, **caractérisé en ce que** dans l'étape a), la température du fond dans le dispositif de distillation (D1) est de 175 à 250°C et/ou **en ce qu'**on élimine par distillation via la tête de (D1), totalement ou du moins en grande partie, à partir du mélange (G1), l'eau qui y est contenue.

10. Procédé selon l'une quelconque des revendications 1 à 9, **caractérisé en ce que** dans l'étape a), le mélange (G2) soutiré du fond du dispositif de distillation (D1) contient moins de 1000 ppm en poids d'eau, plus préférablement moins de 200 ppm en poids d'eau, en particulier moins de 50 ppm en poids d'eau.

11. Procédé selon l'une quelconque des revendications 1 à 10, **caractérisé en ce que** dans l'étape b), le dispositif de distillation (D2) est exploité à une température du fond de 20 à 75°C et/ou à une pression de tête de 10 à 500 mbara, en particulier de 50 à 200 mbara.

12. Procédé selon l'une quelconque des revendications 1 à 11, **caractérisé en ce que** dans l'étape b), le flux (S3) soutiré du fond du dispositif de distillation (D2) contient moins de 10.000 ppm en poids de N-MeEDA, plus préférablement moins de 1000 ppm en poids de N-MeEDA, en particulier moins de 200 ppm en poids de N-MeEDA.

13. Procédé selon l'une quelconque des revendications 1 à 12, **caractérisé en ce que** dans l'étape b), le flux (S2) éliminé par distillation via la tête du dispositif de distillation (D2) contient 20 à 50 ppm en poids d'EDA, plus préférablement 22 à 40 ppm en poids d'EDA, en particulier 23 à 30 ppm en poids d'EDA.

14. Procédé selon l'une quelconque des revendications 1 à 13, **caractérisé en ce que** le mélange (G1) contient essentiellement, de préférence à raison d'au moins 99% en poids, en particulier à raison d'au moins 99,5% en poids, de l'EDA, de la N-MeEDA, de la DETA, de l'eau et du NH₃.

15. Procédé selon l'une quelconque des revendications 1 à 14, **caractérisé en ce que** dans l'étape c), le NH₃ est séparé et recyclé dans une étape de procédé précédente, de préférence, le recyclage d'ammoniac a lieu après l'étape A2) du procédé (A) ou après au moins une des deux étapes B1) ou B2) du procédé (B).
